# EUROPEAN PATENT APPLICATION

(11) **EP 4 523 524 A1**
(43) Date of publication of application: **19.03.2025**
(21) Application number: 22941683.9
(22) Date of filing: 12.05.2022
(51) Int. Cl.: A01K 67/033

(54) **REARING APPARATUS**

(71) Applicant: JTEKT Corporation, Kariya-shi, Aichi 448-8652 (JP)
(72) Inventor: MIURA Nozomu, Kariya-shi, Aichi 448-8652 (JP); NAKAMURA Teruhiro, Kariya-shi, Aichi 448-8652 (JP); FURUYOSHI Masakazu, Kariya-shi, Aichi 448-8652 (JP); MURATA Yasuhiro, Kariya-shi, Aichi 448-8652 (JP); KAWAI Shigekazu, Kariya-shi, Aichi 448-8652 (JP)
(74) Representative: TBK
(86) International application number: PCT/JP2022/020073
(87) International publication number: WO 2023/218603

(57) **Abstract**

A breeding device (1) for breeding a living organism (C) includes a breeding case (10) that has a breeding area (10a) for the living organism (C), and an environment setting unit (30) that sets an environment of the breeding area (10a) of the breeding case (10), in which the environment setting unit (30) is configured to set the environment of at least one of a transporting-direction upstream side region (24) and a transporting-direction downstream side region (25) of the breeding area (10a) to an environment preferred by the living organism (C), based on the nature of the living organism (C), such that relocation of the living organism (C) from the transporting-direction downstream side region (25) to the transporting-direction upstream side region (24) is encouraged.

## Description

### TECHNICAL FIELD

The present disclosure relates to a breeding device.

### BACKGROUND ART

The following Patent Document 1 describes a breeding device for breeding living organisms. This breeding device is equipped with a belt conveyor located at a bottom portion of a breeding case. This belt conveyor has a belt that serves as a floor of the breeding case, and this belt is configured to move in a right-left direction in accordance with rotation of rollers. This belt conveyor enables debris (e.g., droppings, exuviae, carcasses, leftover food, etc.) that has accumulated on the floor of the breeding case to be carried out from inside to outside of the breeding case by rotating the rollers.

### Related Art Documents

### Patent Documents

Patent Document 1: [Patent Document 1] Japanese Unexamined Patent Application Publication No. 2021-151190 (JP 2021-151190 A)

### SUMMARY OF THE INVENTION

In the above breeding device, when the living organisms are staying on the belt, the living organisms are transported from an upstream side of a transporting direction to a downstream side in the transporting direction of the belt, in conjunction with the movement of the belt when cleaning the debris. At this time, the living organisms are disproportionately converged in a transporting-direction downstream side region, and accordingly a breeding concentration of the living organisms in this region rises. High breeding concentration can lead to insufficient food, insufficient water, cannibalism, and so forth, which can be a factor in declining survival rate of the living organisms. Accordingly, when the breeding concentration of living organisms in a predetermined region becomes high, it is necessary to transition this region from an overcrowded state to a sparsely populated state, to lower the breeding concentration of the living organisms. This enables overcrowding of the living organisms in a breeding area to be suppressed, thereby suppressing decline in the survival rate of the living organisms.

Also, in the above breeding device, collection work is performed to collect the living organisms after a breeding period has ended. **In** this collection work, in order to make the work more efficient, it is preferable to gather the living organisms scattered throughout the entire breeding area in a collection region. That is to say, when collecting the living organisms, there is a need to transition the collection region from a sparsely populated state of the living organisms to an overcrowded state thereof, thereby increasing the breeding concentration of the living organisms.

As described above, in a case of taking into consideration both improvement of the survival rate of the living organisms, and efficiency of the collection work, design of this type of breeding device requires technology that enables the breeding concentration of living organisms in each region of the breeding area to be adjusted in accordance with intent of users.

The present disclosure aims to provide a breeding device that is capable of adjusting the breeding concentration of living organisms such as insects or the like.

### Means for Solving the Problem

An aspect of the present disclosure is a breeding device for breeding a living organism includes: a breeding case that has a breeding area for the living organism; and an environment setting unit that sets an environment of the breeding area of the breeding case, in which the environment setting unit is configured to set the environment of at least a first region and a second region of the breeding area to an environment preferred by the living organism, based on nature of the living organism, such that relocation of the living organism from either the first region or the second region to the other is encouraged.

### Effects of the Invention

According to the breeding device of the above aspect, the environment of the breeding area of the breeding case is set by the environment setting unit. The environment setting unit sets the environments of the first region and the second region of the breeding area to be different from each other, based on the nature of the living organism, in order to encourage relocation of the living organism from at least one of the first region and the second region to the other.

Such environment settings by the environment setting unit enables the living organism to be relocated between the two regions by taking advantage of the difference in the environments of the first region and the second region. Setting one of the environment of the first region and the environment of the second region to an environment preferred by the living organism enables the living organism to be easily relocated between the first region and the second region. This enables the breeding concentration of the living organism in each of the first region and the second region to be adjusted. For example, the breeding concentration of the second region can be adjusted to be lowered by relocating the living organism from the second region to the first region, or the breeding concentration of the second region can be adjusted to be raised by relocating the living organism from the first region to the second region.

According to the above aspect, a breeding device can be provided that is capable of adjusting the breeding concentration of living organisms such as insects or the like.

Note that signs in parentheses in the claims indicate corresponding relations with specific means described in the embodiments described below, and are not intended to limit the technical scope of the present disclosure.

### BRIEF DESCRIPTION OF THE DRAWINGS

The foregoing and other objects, features, and advantages of the present disclosure will become more apparent from the following detailed description when taken in conjunction with the accompanying drawings, wherein, in the drawings,
[FIG. 1] FIG. 1 is a perspective view of a breeding device according to a first embodiment, as viewed from diagonally above;
[FIG. 2] FIG. 2 is a frontal view of the breeding device of the first embodiment;
[FIG. 3] FIG. 3 is a perspective view illustrating a state in which a breeding unit in the breeding device of FIG. 1 moves from an initial position to a carry-out preparation position;
[FIG. 4] FIG. 4 is a plan view illustrating a state of an environment setting for encouraging relocation of living organisms during a breeding period in the breeding device of the first embodiment;
[FIG. 5] FIG. 5 is a plan view illustrating a state of an environment setting for encouraging relocation of living organisms during a collecting period in the breeding device of the first embodiment;
[FIG. 6] FIG. 6 is a perspective view of a breeding device according to a second embodiment, as viewed from diagonally above;
[FIG. 7] FIG. 7 is a frontal view of the breeding device of the second embodiment;
[FIG. 8] FIG. 8 is a plan view illustrating a state of an environment setting for encouraging relocation of living organisms during a breeding period in the breeding device of the second embodiment; and
[FIG. 9] FIG. 9 is a plan view illustrating a state of an environment setting for encouraging relocation of living organisms during a collecting period in the breeding device of the second embodiment.

### MODES FOR CARRYING OUT THE INVENTION

A specific structure of a breeding device that is an embodiment of the above aspect will be described below with reference to the drawings.

Note that in the present specification and drawings, unless otherwise specified, a first direction, which is an up-down direction of the breeding device when in a state of having been placed on a horizontal face, is indicated by an arrow X, a second direction, which is a width direction of the breeding device, is indicated by an arrow Y, and a third direction, which is a longitudinal direction of the breeding device, is indicated by an arrow Z.

### (First Embodiment)

### 1. Breeding Objects

Living organisms that are breeding objects are small living organisms such as arthropods, for example. The breeding objects are, for example, living organisms to be used for food, livestock feed, research, and so forth. Examples of the breeding objects can be insects such as crickets, locusts, grasshoppers, and so forth. In particular, the living organisms that are the breeding objects are preferably arthropods that exhibit incomplete metamorphosis, i.e., change directly from nymph state to adult state, and more preferably are nymphs of arthropods that exhibit incomplete metamorphosis. In this example, among insects, nymphs of crickets and locusts of the order Orthoptera will be described as suitable examples of living organisms that are breeding objects. However, the breeding objects may be adults (emerged arthropods with incomplete metamorphose). The breeding device may also be used to breed living organisms from eggs, or to breed hatched living organisms.

### 2. Basic Configuration of Breeding Device

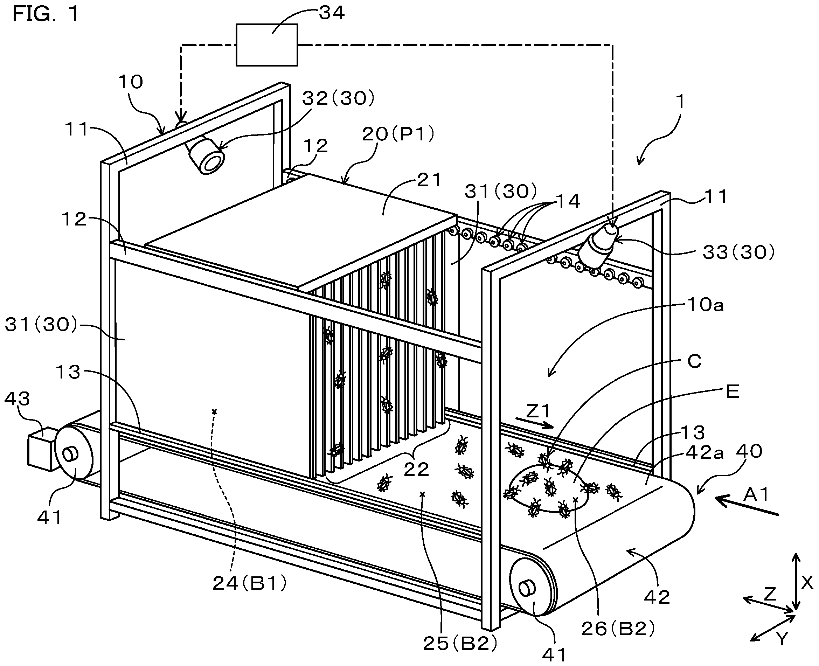

As illustrated in FIG. 1, a breeding device 1 according to a first embodiment is used to breed living organisms C that are breeding objects. In FIG. 1, crickets, which are a type of insect, are exemplified as the living organisms C. The breeding device 1, in a basic configuration thereof, includes a breeding case 10, a breeding unit 20, an environment setting unit 30, and a transporting device 40.

The breeding case 10 is placed on an installation face for the breeding device 1. The breeding case 10 is formed using a plurality of frames. The plurality of frames include two first frames 11 provided on both ends in a third direction Z, two second frames 12 connecting the two first frames 11, and two third frames 13 connecting the two first frames 11. The two second frames 12 extend parallel to each other in the third direction Z, which is the longitudinal direction, and are spaced apart in the second direction Y. The two third frames 13 extend parallel to each other in the third direction Z, which is the longitudinal direction, and are spaced apart in the second direction Y.

The breeding case 10 has a breeding area 10a which serves as a breeding space for the living organisms C. The breeding area 10a is covered by a holding portion 21 located thereabove, and by a plurality of wall portions (omitted from illustration). For this reason, the breeding area 10a is constantly kept as an enclosed space that is enclosed during a breeding period, and this enclosed space is set to an environment suitable for growth of the living organisms C, as appropriate.

### 3. Internal Structure of Breeding Case

The breeding case 10 is configured to accommodate the breeding unit 20 in the breeding area 10a. Note that while FIG. 1 exemplifies a case in which only one breeding unit 20 is accommodated in the breeding area 10a, for the sake of convenience of description, but the number of the breeding unit 20 is not limited to one, and can be changed as appropriate.

The two second frames 12 of the breeding case 10 are support elements for supporting the breeding unit 20 so as to be capable of sliding in the third direction Z. For this reason, a plurality of support rollers 14 are provided along the third direction Z on an inner face of each second frame 12 in the second direction Y. The support rollers 14 are each rotatable members such as rollers or bearings, and are configured to rotate upon receiving input in the third direction Z.

### 4. Structure of Breeding Unit

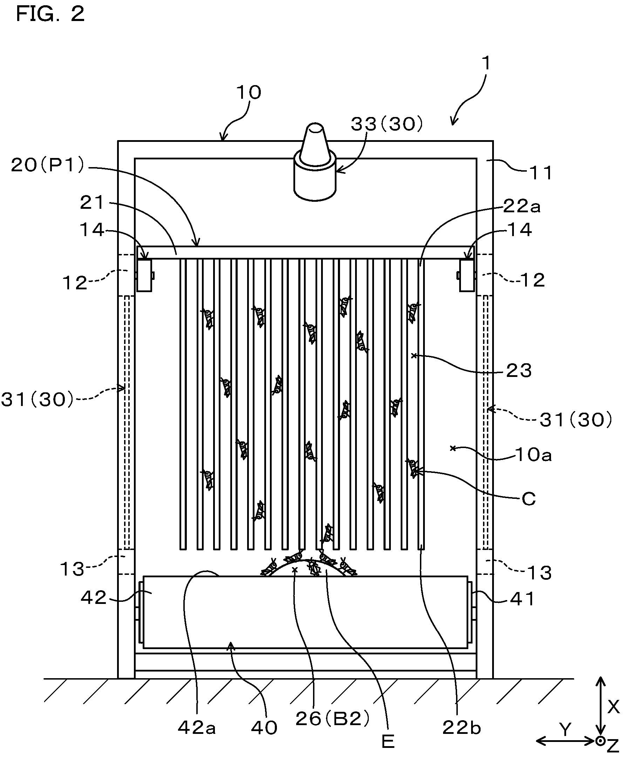

As illustrated in FIG. 1 and FIG. 2, the breeding unit 20 has a holding portion 21 and a plurality of perch members 22.

The holding portion 21 is a plate-shaped member of which a plate thickness direction is in the first direction X. This holding portion 21 is configured to serve as a wall portion (top plate portion) that sections off an upper side of the breeding area 10a. The perch members 22 are for the living organisms C to perch on in the breeding area 10a. The plurality of perch members 22 are fixed to the holding portion 21, and thus are configured to form a hideaway for the living organisms C. For this reason, the breeding unit 20 having the plurality of perch members 22 is also referred to as a "hideaway unit".

As illustrated in FIG. 2, when viewing the breeding device 1 in FIG. 1 from front in a direction of arrow A1, the plurality of perch members 22 extend downward, to lower end portions 22b thereof from an underside of the holding portion 21, with end portions closer to the holding portion 21 serving as basal end portions 22a. The plurality of perch members 22 are arrayed in the second direction Y with gaps 23 of the same dimension therebetween. That is to say, the plurality of perch members 22 are disposed at substantially equidistant intervals in the second direction Y. The gaps 23 are set to dimensions of a size that enable movement of the living organisms C, and also that serve as a hideaway for the living organisms C, in accordance with the physical size thereof.

The perch members 22 are plate-like members that extend in the first direction X along a plane defined by the first direction X and the third direction Z, with the second direction Y being the plate thickness direction. The perch members 22 are configured to enable the living organisms C that are the breeding objects to perch on surfaces thereof. The perch members 22 may be formed in a shape of a flat plate, or may be formed with a corrugated shape. In this example, the perch members 22 are formed in the shape of a flat plate.

The material for the perch members 22 is not limited in particular, and examples of the material that can be used as appropriate include metals such as iron, aluminum, or the like, resin, rubber, wood, paper, and so forth. The perch members 22 preferably have a mesh structure with a great number of through holes formed all over, so as to serve as footholds for the living organisms C. For example, the perch members 22 can be made of punched metal, wire mesh, or the like.

### 5. Structure of Transporting Device

As illustrated in FIG. 1, the breeding device 1 is provided with the transporting device 40 on a floor face of the breeding area 10a of the breeding case 10. The transporting device 40 has a pair of rollers 41, 41 disposed apart from each other in the third direction Z, an endless conveyor belt 42 stretched around the pair of rollers 41, 41, and a motor 43 that drives one of the rollers 41.

In the transporting device 40, when the motor 43 drives one of the rollers 41 and the roller 41 rotates about a rotation axis extending in the second direction Y, a transporting face 42a of the conveyor belt 42 moves continuously in the transporting direction. The transporting face 42a of the conveyor belt 42 forms the floor face (bottom face) of the breeding area 10a.

Here, the transporting direction of the transporting device 40 means a direction in which the transporting face 42a of the conveyor belt 42 moves. The motor 43 and the rollers 41, 41 may be rotatable in just one direction or may be rotatable in both directions. That is to say, in a case in which the motor 43 is rotatable in just one direction, the transporting direction of the transporting device 40 is one direction in the third direction Z. In a case in which the motor 43 is rotatable in both directions, the transporting direction of the transporting device 40 is both directions of the third direction Z.

The transporting device 40 functions to transport at least one of the living organisms C themselves that are the object of breeding, food and water to be given to the living organisms C, exuviae and carcasses of the living organisms C, the living organisms C in early stages of growth or eggs of the living organisms C, droppings, leftover food, and dust. For example, the transporting device 40 can be used to transport food E for breeding, from outside into the breeding area 10a. In this case, a region of the transporting face 42a of the conveyor belt 42 on which food E is placed is a feeding region 26 that is a feeding site for the living organisms C. Note that a watering region that serves as a watering spot for the living organisms C may be provided on the transporting face 42a of the conveyor belt 42, in place of or in addition to the feeding region 26.

### 6. Structure of Environment Setting Unit

As illustrated in FIG. 1, the environment setting unit 30 functions to set a light-dark environment of the breeding area 10a of the breeding case 10. This environment setting unit 30 is configured to encourage relocation of the living organisms C from either a first region B1 or a second region B2 to the other by setting the light-dark environments of the first region B1 and the second region B2 of the breeding area 10a to be different from each other. Note that the entire region of the breeding area 10a may be formed of the first region B1 and the second region B2, or alternatively, the breeding area 10a may include one or a plurality of regions other than the first region B1 and the second region B2.

Here, when the transporting direction of the transporting device 40 is a direction indicated by arrow Z1, and when the breeding unit 20 is at the initial position P1, the transporting-direction upstream side region 24 of the breeding area 10a in the transporting direction by the transporting device 40 (hereinafter simply referred to as the "upstream side region 24") corresponds to the first region B1. Conversely, the transporting-direction downstream side region 25 in the transporting direction by the transporting device 40 (hereinafter simply referred to as the "downstream side region 25") of the breeding area 10a corresponds to the second region B2. Note that the downstream side region 25 also includes the feeding region 26 on the transporting face 42a of the conveyor belt 42. Accordingly, in the present embodiment, the feeding region 26 is also the second region B2, in the same way as the downstream side region 25.

As illustrated in FIG. 1, the environment setting unit 30 includes light blocking members 31, two lighting devices 32 and 33, and a control unit 34 that controls each of the two lighting devices 32 and 33.

The light blocking members 31 are for blocking light from outside the breeding area 10a. Typically, two side wall portions covering the breeding area 10a from both sides in the second direction Y can be made up of the light blocking members 31. In the present embodiment, the light blocking members 31 are attached to the frames 12 and 13 of the breeding case 10 so as to cover the upstream side region 24 from outward sides in the second direction Y. In a state in which the light blocking members 31 are attached to the breeding case 10, the light blocking members 31 block light from entering the upstream side region 24 from the outside, and accordingly the upstream side region 24 is in a darkened state in comparison with the state thereof before attachment of the light blocking members 31.

Note that instead of the present embodiment, the light blocking members 31 may be curtain members such as blackout curtains, and the curtain members may be attached to the outside of side wall portions that are translucent. In a state in which the curtain members are attached, light is blocked from entering the upstream side region 24 from the outside due to light blocking functions of the curtain members, and accordingly the upstream side region 24 is in a darkened state in comparison with the state thereof before attachment of the curtain members. In contrast, in a state in which the curtain members are removed, the upstream side region 24 is in a brightened state since light entering from the outside is not blocked, and entry of light is secured through the side wall portions.

Both the lighting devices 32 and 33 are configured as lighting devices that can be switched between a lit state and an unlit state. The lighting device 32 is disposed facing the upstream side region 24, and by being turned to the lit state by the control unit 34, projects illumination light to the upstream side region 24. This increases the brightness of the upstream side region 24 as compared to a state of receiving only indoor lighting. On the other hand, the lighting device 33 is disposed facing the downstream side region 25, and by being turned to the lit state by the control unit 34, projects illumination light to the downstream side region 25. Thus, the downstream side region 25, like the upstream side region 24, has a higher level of brightness as compared to a state of receiving only indoor lighting.

### 7. Operations of Breeding Unit

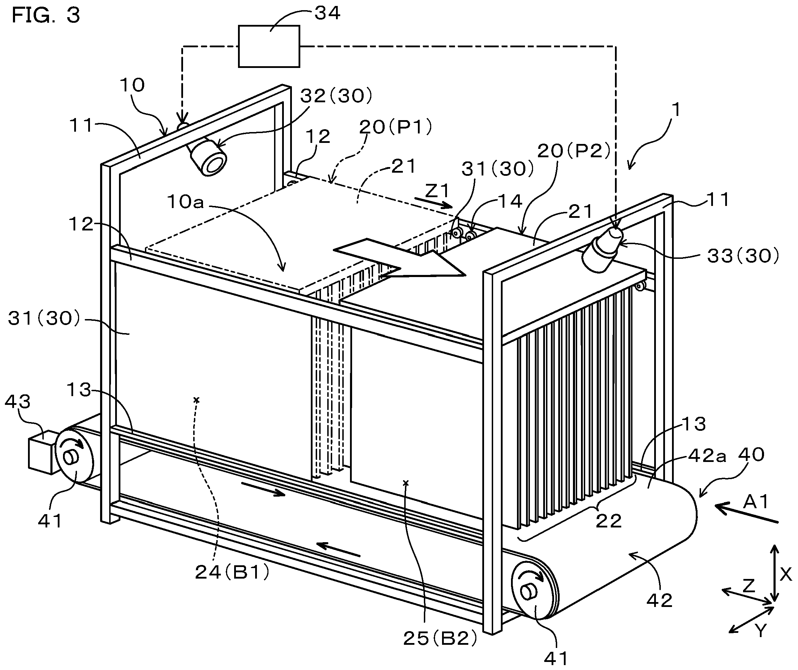

Operations of the breeding unit 20 having the above configuration will be described with reference to FIG. 3.

As illustrated in FIG. 3, living organisms C are bred in the breeding area 10a of the breeding case 10 during the breeding period. At this time, the breeding unit 20 is disposed at the initial position P1. When the breeding period of the living organisms C ends, work transitions to collection work. In this collection work, an operation of sliding the breeding unit 20 in a transporting direction Z1 is executed.

In a case in which the breeding unit 20 does not have self-propelling means, a worker can slide the breeding unit 20 in the transporting direction Z1 by directly grasping the breeding unit 20 with his/her fingers and performing moving thereof manually. In contrast, in a case in which the breeding unit 20 includes driving means, such as an actuator or the like, the breeding unit 20 can be automatically slid in the transporting direction Z1 using the driving means.

The breeding unit 20 slides in the transporting direction Z1 in a state in which both end portions of the holding portion 21 in the third direction Z are supported from below by the plurality of support rollers 14. The breeding unit 20 starts from the initial position P1, passes over a carry-out preparation position P2, and is carried out from the breeding case 10. The living organisms C are then collected from the breeding unit 20 that is carried out.

### 8. Operations of Environment Setting Unit

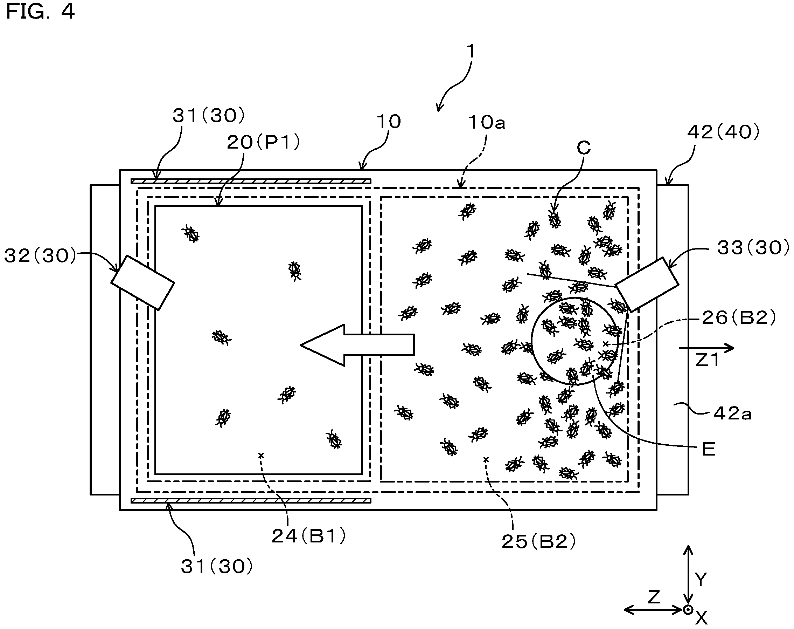

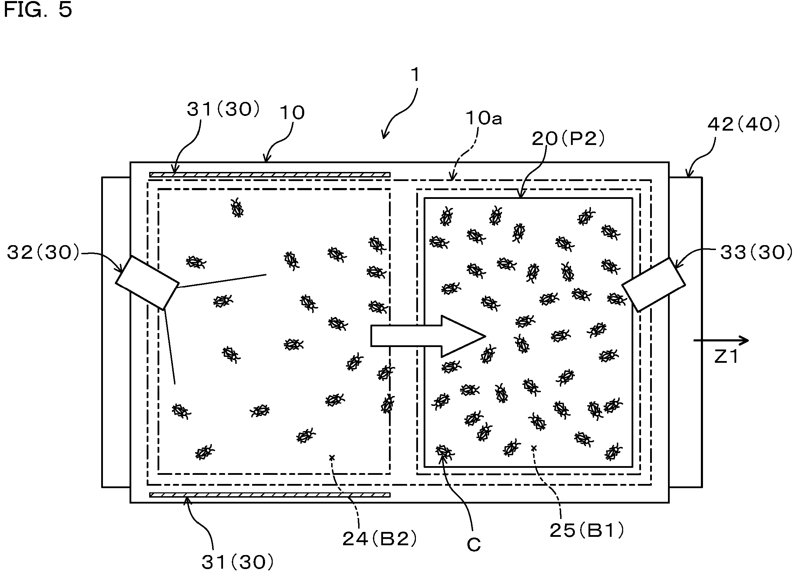

Operations of the environment setting unit 30 having the above configuration will be described with reference to FIG. 4 and FIG. 5.

As illustrated in Figure 4, in a state in which the breeding unit 20 is disposed at the initial position P1 during the breeding period of the living organisms C, the transporting device 40 is driven for the purpose of feeding food E to the living organisms C and for the purpose of carrying out debris. Driving the transporting device 40 causes the transporting face 42a of the conveyor belt 42 to move in the transporting direction Z1.

At this time, the living organisms C staying on the transporting face 42a of the conveyor belt 42 are relocated to the downstream side region 25 as the conveyor belt 42 moves. Accordingly, as the living organisms C become disproportionately converged in the downstream side region 25 within the breeding area 10a, the breeding concentration in the downstream side region 25 becomes locally high. Also, since the living organisms C tend to gather in the feeding region 26, providing the feeding region 26 on the transporting face 42a of the conveyor belt 42 also increases the breeding concentration in the downstream side region 25. On the other hand, the upstream side region 24 tends to be in a sparsely populated state with a low breeding concentration of living organisms C due to the disproportionate convergence of living organisms C in the downstream side region 25. Furthermore, when the downstream side region 25 is in an overcrowded state with the living organisms C at a high breeding concentration, food shortages, water shortages, cannibalism, and so forth will be induced, which can result in a decline in the survival rate of the living organisms C.

The present embodiment takes advantage of the fact that cricket-like living organisms C are nocturnal, and have nature of preferring darkness and disliking brightness. During the breeding period of the living organisms C, the upstream side region 24 is set as the first region B1 and the downstream side region 25 is set as the second region B2, and the environment setting unit 30 sets the environment so as to encourage relocation of the living organisms C from the second region B2 to the first region B1.

Based on the above nature of the living organisms C, the environment setting unit 30 sets the upstream side region 24 to a favorable environment preferred by the living organisms C when the breeding unit 20 is at the initial position P1. That is to say, the environment setting unit 30 uses the light blocking members 31 to perform environment settings such that the upstream side region 24 is darker as compared to the downstream side region 25. Such environmental settings can encourage the relocation of the living organisms C that prefer darkness from the downstream side region 25 to the upstream side region 24.

Accordingly, the living organisms C that have been relocated to the downstream side region 25 along with the movement of the conveyor belt 42, and the living organisms C that have finished feeding in the feeding region 26, relocate to the upstream side region 24, which is dark, in accordance with their nature. As a result, the breeding concentration of the living organisms C in the downstream side region 25 can be adjusted so as to be lowered during the breeding period of the living organisms C, and the locally overcrowded state of the living organisms C created in the downstream side region 25 can be resolved. Accordingly, the environmental function provided by the environment setting unit 30 can also be called a breeding concentration adjustment function for adjusting the breeding concentration of the living organisms C in the breeding area 10a.

Note that the lighting device 33 can be used in addition to the light blocking members 31, as necessary. The lighting device 33 is turned to the lit state by the control unit 34. Accordingly, in addition to setting the upstream side region 24 to a favorable environment preferred by the living organisms C, the downstream side region 25 can be set to an unfavorable environment disliked by the living organisms C as compared to the upstream side region 24.

According to such environmental settings, the living organisms C in the downstream side region 25 relocate toward the darkness of the upstream side region 24 and also relocate to escape into the upstream side region 24 and avoid the illumination light from the lighting device 33. Accordingly, the effects of encouraging the relocation of the living organisms C from the downstream side region 25 to the upstream side region 24 are enhanced. As a result, the locally overcrowded state of the living organisms C in the downstream side region 25 can be resolved in a short period of time.

Note that as a modification of the present embodiment, the relocation of the living organisms C from the downstream side region 25 to the upstream side region 24 may be encouraged without using the light blocking members 31. In this case, the lighting device 32 is kept in the unlit state and the lighting device 33 is turned to the lit state. This enables the downstream side region 25 to be set to an unfavorable environment that is more disliked by the living organisms C than the upstream side region 24, thereby encouraging the relocation of the living organisms C from the downstream side region 25 to the upstream side region 24.

As illustrated in FIG. 4 and FIG. 5, the breeding unit 20 is disposed at the initial position P1 (see FIG. 4) during the breeding period of the living organisms C, while being disposed at the carry-out preparation position P2 (see FIG. 5) during the collection work of the living organisms C. In other words, the breeding unit 20 is configured to be disposed at different positions in the breeding area 10a during the breeding period of the living organisms C and during the collection work thereof. At this time, the breeding unit 20 is disposed in the upstream side region 24 at the initial position P1, and conversely is disposed in the downstream side region 25 at the carry-out preparation position P2.

Now, when collecting the living organisms C, the living organisms C scattered throughout the breeding area 10a are gathered in the downstream side region 25, enabling the collection work to be performed efficiently in a short amount of time. To achieve this, it is necessary to quickly relocate the living organisms C in the breeding area 10a to the downstream side region 25.

Accordingly, during the collection work of the living organisms C, the downstream side region 25 is set as the first region B1 and the upstream side region 24 is set as the second region B2, which is the opposite to in the breeding period of the living organisms C, and the environment setting unit 30 sets the environment so as to encourage the relocation of the living organisms C from the second region B2 to the first region B1.

Specifically, when the breeding unit 20 is at the carry-out preparation position P2, the environment setting unit 30 causes the control unit 34 to turn the lighting device 32 to the lit state and also causes the control unit 34 to turn the lighting device 33 to the unlit state. This enables the relocation of the living organisms C from the upstream side region 24 to the downstream side region 25 to be encouraged by setting the upstream side region 24 to an unfavorable environment that the living organisms C dislike. As a result, the living organisms C can be locally gathered in the downstream side region 25 during the collection work of the living organisms C. Thereafter, the breeding unit 20, which is at the carry-out preparation position P2, is carried out from the breeding case 10, and thus the living organisms C can be collected.

Next, the functions and effects of the above first embodiment will be described.

According to the breeding device 1 of the first embodiment, the environment of the breeding area 10a of the breeding case 10 is set by the environment setting unit 30. The environment setting unit 30 performs settings such that the environments of the upstream side region 24 and the downstream side region 25 of the breeding area 10a are different from each other, based on the nature of the living organisms C, to encourage the living organisms C to relocate from one to the other of the upstream side region 24 and the downstream side region 25. That is to say, during the breeding period of the living organisms C, the environment is set to encourage the relocation of the living organisms C from the downstream side region 25 to the upstream side region 24, and when the living organisms C are to be collected, the environment is set to encourage the relocation of the living organisms C from the upstream side region 24 to the downstream side region 25.

Such environment settings by the environment setting unit 30 enables the living organisms C to be relocated between the two regions by taking advantage of the difference in the environments of the upstream side region 24 and the downstream side region 25. By setting either the environment of the upstream side region 24 or the environment of the downstream side region 25 to an environment preferred by the living organisms C, the living organisms C can be easily relocated between the upstream side region 24 and the downstream side region 25. Accordingly, the breeding concentration of the living organisms C in each of the upstream side region 24 and the downstream side region 25 can be adjusted. That is to say, the breeding concentration in the downstream side region 25 can be adjusted to be lowered by relocation of the living organisms C from the downstream side region 25 to the upstream side region 24, and the breeding concentration in the downstream side region 25 can be adjusted to be higher by relocation of the living organisms C from the upstream side region 24 to the downstream side region 25. Consequently, such adjustment enables reducing the breeding concentration in the downstream side region 25 to a value lower than the breeding concentration in the upstream side region 24, increasing the breeding concentration thereof to a level higher than that of the upstream side region 24, or equalizing the breeding concentration thereof to be approximately the same as that of the upstream side region 24.

According to the present embodiment, during the breeding period of the living organisms C, overcrowding of the living organisms C in the downstream side region 25 of the breeding area 10a can be suppressed from occurring, and accordingly decline in the survival rate of the living organisms C due to factors such as food shortages, water shortages, cannibalism, and so forth, can be suppressed. On the other hand, the living organisms C can be gathered in the downstream side region 25 of the breeding area 10a when collecting the living organisms C, and accordingly the collection work of the living organisms C can be executed efficiently in a short time.

As described above, according to the first embodiment, the breeding device 1 can be provided that can adjust the breeding concentration of living organisms C, such as insects, taking into consideration both improving the survival rate of the living organisms C, and improving the efficiency of collection work.

Other embodiments related to the first embodiment described above will be described below with reference to the drawings. In other embodiments, elements that are the same as those of the first embodiment are denoted by the same signs thereof, and description of the same elements will be omitted.

### (Embodiment 2)

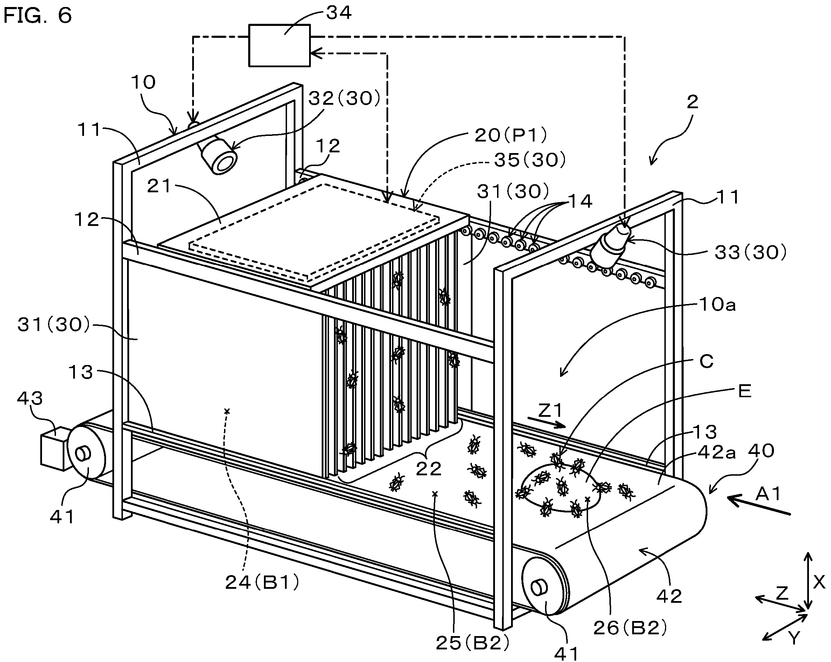

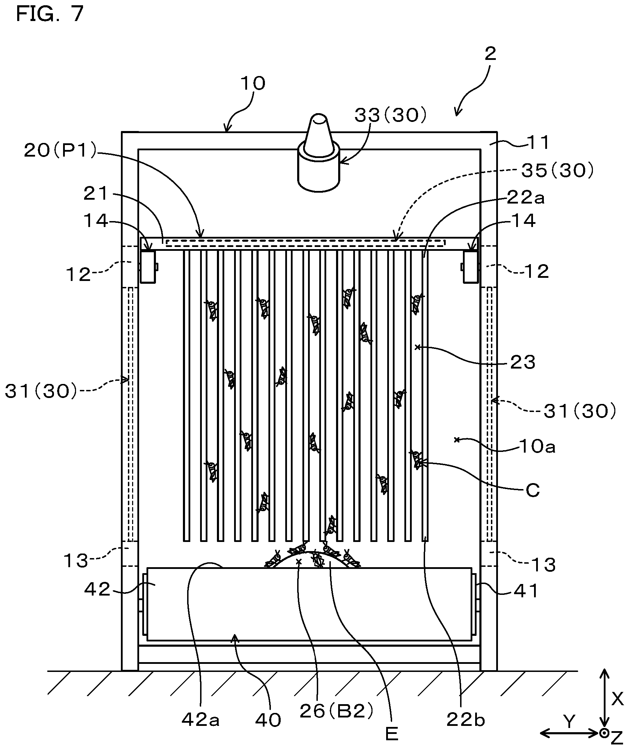

As illustrated in FIG. 6 and FIG. 7, a breeding device 2 according to a second embodiment differs from the breeding device 1 according to the first embodiment with respect to a point that a heater 35 is added to the components of the environment setting unit 30.

The present embodiment takes advantage of the fact that the living organisms C such as crickets are nocturnal, and furthermore have a nature of preferring warm places and disliking cold places. The environment setting unit 30 is provided with the heater 35 to deal with such nature of the living organisms C. This heater 35 is built into the holding portion 21 of the breeding unit 20 and is configured such that output thereof is controlled by the control unit 34. Heat generated by the heater 35 warms the holding portion 21, and further, the heat of the holding portion 21 is conveyed to each perch member 22, such that the overall breeding unit 20 is heated. When the breeding unit 20 is heated, the temperature in the region around the breeding unit 20 in the breeding area 10a becomes locally high.

Other configurations of the breeding device 2 are the same as those of the first embodiment.

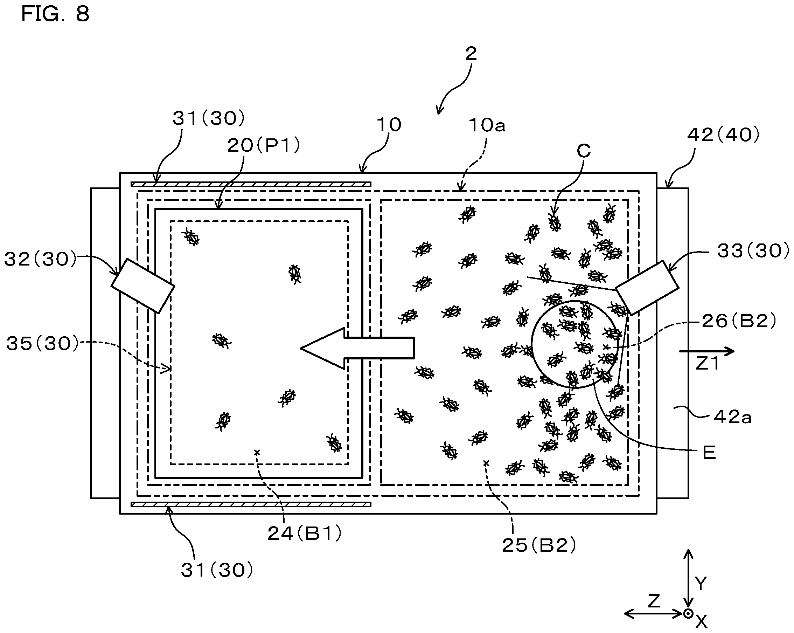

As illustrated in FIG. 8, when the breeding unit 20 is at the initial position P1, the environment setting unit 30 sets the upstream side region 24 to a favorable environment preferred by the living organisms C. That is to say, the environment setting unit 30 sets the environment by operating the heater 35 such that the upstream side region 24 becomes warmer than the downstream side region 25. This can encourage the relocation of the living organisms C, which like warmth, from the downstream side region 25 to the upstream side region 24. As a result, the locally overcrowded state of the living organisms C that occurs in the downstream side region 25 during the breeding period of the living organisms C can be resolved.

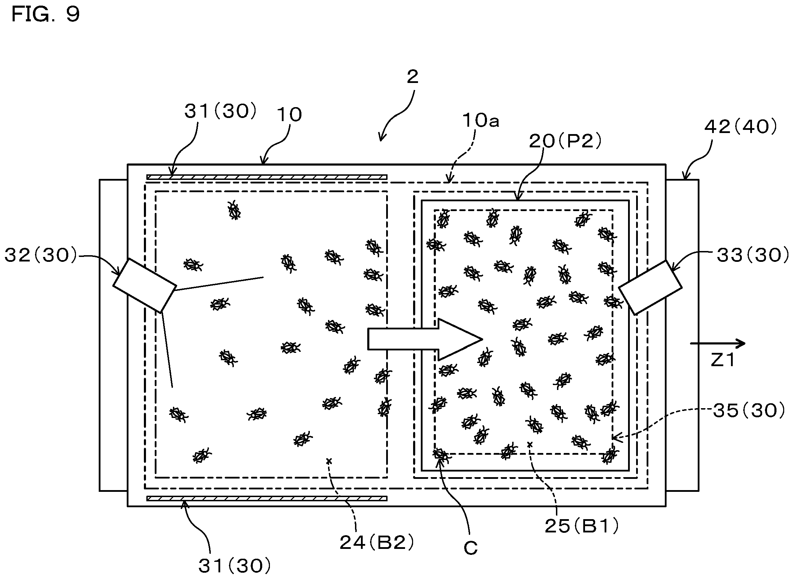

As illustrated in FIG. 9, when the breeding unit 20 is at the carry-out preparation position P2, the environment setting unit 30 sets the downstream side region 25 to a favorable environment preferred by the living organisms C. **In** other words, the environment setting unit 30 sets the environment such that the downstream side region 25 in which the breeding unit 20 is disposed is warmer than the upstream side region 24 by maintaining the operation of the heater 35. This can encourage the relocation of the living organisms C, which like warmth, from the upstream side region 24 to the downstream side region 25. At this time, the living organisms C remaining in the breeding unit 20, which is a warm place, automatically relocate from the upstream side region 24 to the downstream side region 25 as the breeding unit 20 moves. As a result, the living organisms C can be gathered in the downstream side region 25 during the collection work of the living organisms C.

The functions and effects are otherwise the same as those of the first embodiment.

Note that, in a modification example particularly related to the second embodiment, instead of or in addition to the heater 35, a fan capable of blowing out warm air can be employed to create a favorable environment for the living organisms C, or a fan capable of blowing out cold air can be employed to create an unfavorable environment for the living organisms C.

**In** a further modification of the second embodiment, the environment setting unit 30 can be configured based solely on the nature of the living organisms C of preferring warm places and disliking cold places. **In** this case, the light blocking members 31 and the two lighting devices 32 and 33 can be omitted from the components of the environment setting unit 30, and a structure in which only the heater 35 is used can be employed.

While the present disclosure has been described based on the embodiments, it should be understood that the present disclosure is not limited to such embodiments or structures. The present disclosure includes various changes and modifications within the range of equivalency. **In** addition, various combinations and forms, and other combinations and forms including only one element, two or more elements, or fewer elements are encompassed by the spirit and scope of the present disclosure.

**In** the above-described embodiment, a case has been exemplified in which the environment setting unit 30 differs the environment between the upstream side region 24 and the downstream side region 25 of the breeding area 10a, but instead of this, a first region and a second region arrayed in the first direction X, which is the up-down direction, of the breeding area 10a, may be provided and the environments of these two areas may be made different by the environment setting unit 30, or a first area and a second area arrayed in the second direction Y may be provided and the environments of these two areas may be made different by the environment setting unit 30. **In** the case of the first region and the second region in the first direction X, for example, when the breeding concentration of the living organisms C becomes high in a lower region (first region) of the breeding area 10a, the environment can be set such that the relocation of the living organisms C to an upper region (second region) is encouraged by darkening the upper region of the breeding area 10a. Also, when the breeding concentration of the living organisms C becomes high in the lower region of the breeding area 10a, the environment can be set to warm the upper region of the breeding area 10a in order to encourage the relocation of the living organisms C to the upper region.

**In** the above embodiment, a case is exemplified in which the environment set by the environment setting unit 30 is a light-dark environment and a temperature environment, but the environment is not limited to only a light-dark environment and a temperature environment. The environment set by the environment setting unit 30 is selected as appropriate depending on the nature of the living organisms C that are the breeding object. Examples of environments other than the light-dark environment and temperature environment include humidity environment, airflow environment, air pressure environment, feeding environment, watering environment, cleaning environment, and so forth.

Regarding the humidity environment, for example, a humidity range preferred by the living organisms C can be defined as being a favorable environment, and any other humidity range can be defined as being an unfavorable environment. Regarding the airflow environment, for example, an environment in which no airflow is provided to the living organisms C can be defined as being a favorable environment, and an environment in which an airflow is provided to the living organisms C can be defined as being an unfavorable environment. Regarding the air pressure environment, for example, an air pressure range preferred by the living organisms C can be defined as being a favorable environment, and any other air pressure range can be defined as being an unfavorable environment. Regarding the feeding environment, an environment in which a feeding site for the living organisms C is provided in a predetermined region can be defined as being a favorable environment, and an environment in which the feeding site for the living organisms C is provided in any other region can be defined as being an unfavorable environment. Regarding the watering environment, an environment in which a watering spot for the living organisms C is provided in a predetermined region can be defined as being a favorable environment, and an environment in which the watering spot for the living organisms C is provided in any other region can be defined as being an unfavorable environment. Regarding the cleaning environment, for example, an environment in which cleaning work is performed periodically can be defined as being a favorable environment, and an environment in which cleaning work is not performed can be defined as being an unfavorable environment.

In the above embodiment, a case is exemplified in which the transporting device 40 is provided on the floor face of the breeding area 10a of the breeding case 10, but a structure in which the transporting device 40 is omitted can also be employed.

Also, in a case of taking the above embodiments and the description of various modifications into consideration, the following form may also be adopted.

### Form 1

"A method for breeding a living organism (C), wherein
an environment of a first region (B1) and a second region (B2) of a breeding area for the living organism are set to be different from each other, based on nature of the living organism, such that the living organism is encouraged to relocate from one to the other of the first region and the second region".

According to this Form 1, a breeding method can be provided that can adjust the breeding concentration of the living organisms C, such as insects, taking into consideration both improving the survival rate of the living organisms C, and improving the efficiency of collection work.

## Claims

1. A breeding device (1, 2) for breeding a living organism (C), the breeding device comprising:
a breeding case (10) that has a breeding area (10a) for the living organism; and
an environment setting unit (30) that sets an environment of the breeding area of the breeding case, wherein the environment setting unit is configured to set the environment of at least a first region (B1) and a second region (B2) of the breeding area to an environment preferred by the living organism, based on nature of the living organism, such that relocation of the living organism from either the first region or the second region to the other is encouraged.

2. The breeding device according to claim 1, further comprising a transporting device (40) provided on a floor face of the breeding area of the breeding case, wherein:
the breeding area includes a transporting-direction upstream side region (24) in a transporting direction by the transporting device and a transporting-direction downstream side region (25) in the transporting direction by the transporting device; and
the transporting-direction upstream side region is the first region and the transporting-direction downstream side region is the second region.

3. The breeding device according to claim 1 or 2, further comprising a breeding unit (20) having a perch member (22) for the living organism to perch on, wherein:
the breeding area includes a feeding region (26) that serves as a feeding site for the living organism; and
a region in which the breeding unit is disposed during a breeding period of the living organism is the first region and the feeding region is the second region.

4. The breeding device according to claim 3, wherein:
the breeding unit is configured to be disposed at different positions in the breeding area, depending on whether during the breeding period of the living organism or during collection work of the living organism; and
the region in which the breeding unit is disposed during the collection work of the living organism is the first region.

5. The breeding device according to claim 1 or 2, wherein the environment is at least one selected from a light-dark environment, a temperature environment, a humidity environment, an airflow environment, an air pressure environment, a feeding environment, a watering environment, and a cleaning environment.
